# EUROPEAN PATENT APPLICATION

(11) **EP 4 269 459 A1**
(43) Date of publication of application: **01.11.2023**
(21) Application number: 23169765.7
(22) Date of filing: 25.04.2023
(51) Int. Cl.: C08F 290/06, C08G 18/48, C08G 18/67, C08G 18/75, C08G 18/81, C09D 175/16, A61B 5/145

(54) **HYDROPHILIC POLY(METH)ACRYLATE COPOLYMER FILM FOR BIOLOGICAL TEST STRIPS, PREPARATION METHOD AND APPLICATION THEREOF**

(30) Priority: 29.04.2022 US 202263336361 P
(71) Applicant: Covestro (Taiwan) Ltd, Taoyuan City 320317 (TW); Industrial Technology Research Institute, 31040 Hsinchu (TW)
(72) Inventor: CHANG, Jui-Ming, 320317 Taoyuan City (TW); SHIH, Che-Hsiu, 320317 Taoyuan City (TW); YEH, Shu-Ling, Zhubei City (TW); LIN, Yen-Chen, Hsinchu City (TW)
(74) Representative: Levpat

(57) **Abstract**

The present disclosure provides a hydrophilic poly(meth)acrylate copolymer film for biological test strip, a preparation method thereof and a biological test strip containing the hydrophilic poly(meth)acrylate copolymer film. The hydrophilic poly(meth)acrylate copolymer film includes a substrate and a poly(meth)acrylate copolymer formed on at least one surface of the substrate. The poly(meth)acrylate copolymer is derived from a formulation including an acrylate-functioned oligomer and a (meth)acrylic acid or (meth)acrylate monomer; and an initiator.

## Description

### Technical Field

The present disclosure relates to a hydrophilic poly(meth)acrylate copolymer film for biological test strip, a preparation method thereof and a biological test strip containing the hydrophilic poly(meth)acrylate copolymer film.

### Description of the Related Art

In vitro diagnostic devices refer to instruments and systems that use body samples such as blood, urine, stool, saliva, other body fluids or tissue sections to diagnose or monitor diseases, disorders or conditions. Among them, blood glucose meters are widely used by people with diabetes to measure and display the amount of glucose in their blood. Generally, blood sugar is measured by the following steps: inserting a blood glucose test strip into the blood glucose meter, pricking one's fingertip with a tiny needle (lancet), squeezing out a drop of blood and touching the same with the edge of the test strip. The test strip is usually a plastic strip coated with a patterned layer of gold that becomes the strip's circuit. One end of the strip has a coating of chemicals, which soaks up the blood and allows it to flow into a test area (such as an enzyme layer), where the glucose in the blood is turned into electricity for measurement.

Given the rising prevalence of diabetes, the market for blood glucose meters has gained increased attention from manufactures seeking to offer better performing blood glucose strips in terms of speed and accuracy of measurement and stability. The coating of chemicals plays an important role not only in protecting the test area underneath from contamination or storage condition, but also delivering the blood to the test area at an acceptable rate. It is known that the coating of chemicals is preferably hydrophilic to be compatible with blood. However, the hydrophilicity of a hydrophilic coating in the commercial blood glucose test strips is achieved by a surfactant additive. However, the surfactant additive and the resin used for the hydrophilic coating are liable to form a heterogeneous mixture even if they are mixed evenly, which may likely affect the surface roughness, haze, and transmission of the film. Furthermore, the surfactant additive is more prone to be affected by ambient conditions due to its relatively small molecular weight or the lack of strong chemical bonding. It is also possible that the surfactant additive oozes from the resin over time. In addition, the adhesion and environmental resistance of the hydrophilic film to a substrate may decrease when the amount of surfactant additive increases. Accordingly, the amount of surfactant additive added should be kept low, which in turn limits the hydrophilicity.

There is a need for a hydrophilic film that has improved hydrophilicity and stability for use in a blood glucose test strip and other biological test strips.

### SUMMARY

In order to solve the above problems, the inventors have found after repeated studies a poly(meth)acrylate copolymer film which is hydrophilic and needs no surfactants as additives.

The present disclosure provides a hydrophilic poly(meth)acrylate copolymer film, especially a hydrophilic poly(meth)acrylate copolymer film for biological test strip, comprising:
a substrate; and
a poly(meth)acrylate copolymer formed on at least one surface of the substrate; wherein the poly(meth)acrylate copolymer is derived from a formulation comprising:
   an acrylate-functioned oligomer;
   a (meth)acrylic acid or (meth)acrylate monomer; and
   an initiator.

The present disclosure further provides a method of preparing the hydrophilic poly(meth)acrylate copolymer film.

The present disclosure also provides a biological test strip containing the hydrophilic poly(meth)acrylate copolymer film thus prepared.

### DETAILED DESCRIPTION

The contents of the present disclosure will be described in detail below.

As used in the present disclosure, the term "polymer" refers to a compound prepared by polymerizing monomers, whether the same or a different type of monomer is used. The generic term "polymer" may include the terms "oligomer," "homopolymer," "copolymer" or the like.

As used in the present disclosure, the term "homopolymer" refers to polymers prepared by the polymerization of the same type of monomers.

As used in the present disclosure, the term "copolymer" refers to polymers prepared by the polymerization of at least two different types of monomers.

As used in the present disclosure, the term "oligomer" refers to polymers having limited monomer units, such as a dimer, trimer or a polymer having 10 to 100 repetitive units (e.g., 10, 20, 30, 40, 50, 60, 70, 80, 90 or 100 repetitive units) and/or 20 to 200 carbon atoms (e.g., 20, 40, 60, 80, 100, 120, 140, 160, 180 or 200 carbon atoms), in the backbone of the polymer, but is not limited thereto. In some embodiments, the term "oligomer" refers to a pre-synthesized polymer which may be further polymerized with other monomers or oligomers.

### [Poly(meth)acrylate Copolymer]

The poly(meth)acrylate copolymer according to the present disclosure is hydrophilic itself and the hydrophilicity is not achieved by the addition of a surfactant additive. This is achieved by the introduction of hydrophilic segments or hydrophilic groups into the backbone or the end of the poly(meth)acrylate copolymer.

As will be explained below, the poly(meth)acrylate copolymer according to the present disclosure is prepared from a formulation comprising: one or more pre-synthesized acrylate-functioned oligomers, one or more (meth)acrylic acids or (meth)acrylate monomers, and one or more initiators. Contrary to a one-pot reaction where all the starting materials are mixed and reacted together, with the use of a pre-synthesized acrylate-functioned oligomer, it is able to obtain an acrylate-functioned oligomer with desired properties by adjusting/controlling the parameters or steps of the manufacture process thereof, and as a result, it is easier to optimize the final properties of the poly(meth)acrylate copolymer.

### [Acrylate-functioned Oligomer]

The acrylate-functioned oligomer of the present disclosure may be an acrylic acrylate oligomer, epoxy acrylate oligomer, polyester acrylate oligomer or urethane acrylate oligomer. These oligomers may be linear, branched, comb-like, star-shaped, etc. depending on the reactants used and be end-capped with acrylates for subsequent polymerization.

In one embodiment, the acrylate-functioned oligomer is a urethane acrylate oligomer, which may be derived from: a polyol, an isocyanate compound; and a hydroxyl-functioned (meth)acrylate. In this embodiment, the hydroxyl-functioned (meth)acrylate is reacted with an isocyanate compound to form urethane bonds, and the equivalent of the isocyanate group should be more than that of the hydroxyl group, such that the remaining isocyanate group can be reacted with the polyol to form the acrylate-functioned oligomer. The quantity of isocyanate (NCO) content is measured by a potentiometric titrator to monitor the degree of reaction. Generally, the oligomer with a NCO content of <0.1% is considered sufficiently reacted.

In one embodiment, the polyol may be polyether polyol or polyester polyol. The polyether polyol may be ethylene oxide (EO)-based, propylene oxide (PO)-based, or a copolymer of EO and PO. For the copolymer of EO and PO, the content ratio of EO to PO can be any suitable value, such as 10:90, 20:80, 25:75, 30:70, 40:60, 50:50, 60:40, 70:30, 75:25, 80:20 or 90:10. In one embodiment, the polyol has a weight average molecular weight (Mw) of 200 to 10,000, e.g., 200, 400, 600, 800, 930, 1,000, 2,000, 3,000, 3,400, 4,000, 5,000, 6,000, 7,000, 8,000, 9,000 or 10,000. Preferably, the polyol is a poly(ethylene glycol) having a weight average molecular weight of 2,000 to 6,000. The hydrophilicity and viscosity of the acrylate-functioned oligomer may depend on the weight average molecular weight of the polyol or the amount of the EO and/or PO units. The higher the weight average molecular weight of the polyol or the amount of the EO and/or PO units, the higher its hydrophilicity and viscosity.

In one embodiment, the isocyanate compound may be aliphatic isocyanate, cycloaliphatic isocyanate, araliphatic isocyanate, aromatic isocyanate, or a combination thereof. In one embodiment, the isocyanate compound contains two to six isocyanate groups, e.g., two, three, four, five or six isocyanate groups. If the isocyanate compound contains more than two isocyanate groups, a branched oligomer can be expected. In one embodiment, the molecular weight of the isocyanate compound ranges from 100 to 500, such as 100, 200, 300, 400 or 500.

Examples of isocyanate compounds include (but are not limited to) 1,4-diisocyanatobutane, 1,6-hexanediisocyanate (HDI), 2-methyl-1,5-diisocyanatopentane, 1,5-diisocyanato-2,2-dimethyl pentane, 2,2,4-trimethyl-1,6-diisocyanatohexane, 2,4,4-trimethyl-1,6-diisocyanatohexane, 1,10-diisocyanatodecane, 1,3-diisocyanatocyclohexane, 1,4-diisocyanatocyclohexane, 1,3-bis(isocyanatomethyl)-cyclohexane, 1,4-bis(isocyanatomethyl)-cyclohexane, isophorone diisocyanate (IPDI), 4,4'-diisocyanatodicyclohexylmethane (H12MDI), 1-isocyanato-1-methyl-4(3)-isocyanato-methylcyclohexane, bis(isocyanatomethyl)-norbomane, 1,3-bis-(2-isocyanato-prop-2-yl)-benzene, 1,4-bis-(2-isocyanato-prop-2-yl)-benzene, 2,4-diisocyanatotoluene, 2,6-diisocyanatotoluene (TDI), 2,4'-diisocyanatodiphenylmethane, 4,4'-diisocyanatodiphenylmethane (MDI) and 1,5-diisocyanatonaphthalene or a mixture thereof. In one embodiment, the isocyanate compound is isophorone diisocyanate (IPDI). In another embodiment, the isocyanate compound is 4,4'-diisocyanatodicyclohexylmethane (H12MDI).

In one embodiment, the hydroxyl-functioned (meth)acrylate is a hydroxyl-substituted C₁₋₆alkyl (meth)acrylate, e.g., hydroxyl-substituted C₁alkyl (meth)acrylate, hydroxyl-substituted C₂alkyl (meth)acrylate, hydroxyl-substituted C₃alkyl (meth)acrylate, hydroxyl-substituted C₄alkyl (meth)acrylate, hydroxyl-substituted C₅alkyl (meth)acrylate or hydroxyl-substituted C₆alkyl (meth)acrylate. In one embodiment, the hydroxyl-functioned (meth)acrylate is 2-hydroxyethyl methacrylate (HEMA). In another embodiment, the hydroxyl-functioned (meth)acrylate is 2-hydroxyethyl acrylate (HEA). Preferably, the hydroxyl-functioned (meth)acrylate is 2-hydroxyethyl methacrylate (HEMA).

In one embodiment, the acrylate-functioned oligomer is a linear urethane acrylate oligomer. This is achieved by reacting a diisocyanate with a hydroxyl-functioned (meth)acrylate, followed by reacting with a linear, bifunctional polyol. The resulting oligomer has the following structure: (meth)acrylate-diisocyanate-polyoldiisocyanate-(meth)acrylate. In a preferred embodiment, the acrylate-functioned oligomer is a linear urethane acrylate oligomer having the following structure: methacrylate-diisocyanate-poly(ethylene glycol)-diisocyanate-methacrylate.

In one embodiment, additives may be added for preparing the acrylate-functioned oligomer. The additives are not particularly limited and can be any suitable additives known by those skilled in the art. In one embodiment, the additive may include an antioxidant and/or catalyst in an amount of 5 wt% or less, e.g., 5 wt%, 4 wt%, 3 wt%, 2 wt%, 1 wt%, 0.5 wt%, 0.05 wt% or 0.01 wt%, based on the total weight of the acrylate-functioned oligomer. The antioxidant, including (but not limited to) butylated hydroxytoluene (BHT) and butylated hydroxyanisole (BHA), is used as a preservative. The catalyst, such as bismuth carboxylate, is used to facilitate the formation reaction of the acrylate-functioned oligomer.

### [(Meth)acrylic Acid or (Meth)acrylate Monomer]

The (meth)acrylic acid of the present disclosure may be methacrylic acid or acrylic acid, which contributes a carboxyl group to the poly(meth)acrylate copolymer such that the hydrophilicity is enhanced.

The (meth)acrylate monomer of the present disclosure may be C₁₋₁₅₀alkyl (e.g., C₁₋₁₀₀alkyl, C₁₋₅₀alkyl, C₁₋₁₀alkyl or C₁₋₅alkyl) (meth)acrylate optionally substituted with one or more functional groups, wherein the alkyl may be linear, branched or cyclic. In some embodiments, the (meth)acrylate monomer comprises an ionic (meth)acrylate monomer or an non-ionic (meth)acrylate monomer. In some embodiments, the ionic (meth)acrylate monomer is cationic, anionic or amphoteric. In some embodiments, the (meth)acrylate monomer bears a hydroxyl, carboxylate, phosphate, sulfate or ammonium group, or an amino acid as functional group(s). In one embodiment, the (meth)acrylate monomer has a hydroxyl group. In another embodiment, the (meth)acrylate monomer has a phosphate group. In yet another embodiment, the (meth)acrylate monomer has a sulfate group.

In some embodiments, the (meth)acrylate monomer contains a hydrophilic repeating unit. In some embodiments, the hydrophilic repeating unit is -(O-CH₂-CH₂)ₙ-, wherein n is 1 to 50, e.g., 1, 2, 3, 4, 5, 6, 7, 8, 9, 10, 12, 14, 15, 16, 18, 20, 25, 30, 35, 40, 45 or 50. Preferably, the (meth)acrylate monomer contains a hydrophilic repeating unit -(O-CH₂-CH₂)ₙ- with n being 2 to 6 and has a sulfate group.

In some embodiments, the (meth)acrylate monomer comprises a (meth)acrylate crosslinker. As used herein, the term "crosslinker" refers to a molecule comprising two or more (meth)acrylate groups, e.g., 2, 3, 4, 5, 6, 7, 8, 9, 10, 11, 12, 13, 14 or 15 (meth)acrylate groups, that are reactive with other (meth)acrylate groups and capable of linking two or more monomers or oligomers through chemical bonds.

Examples of the (meth)acrylate monomers include (but are not limited to) methyl (meth)acrylate, ethyl (meth)acrylate, propyl (meth)acrylate, n-butyl (meth)acrylate, 2-ethylhexyl (meth)acrylate, hexyl (meth)acrylate, amyl (meth)acrylate, heptyl (meth)acrylate, n-octyl acrylate, n-octyl methacrylate, isooctyl (meth)acrylate, isononyl (meth)acrylate, isobutyl (meth)acrylate, 2-butyl (meth)acrylate, decyl (meth)acrylate, isodecyl (meth)acrylate, isotridecyl (meth)acrylate, benzyl (meth)acrylate, lauryl (meth)acrylate, octadecyl (meth)acrylate, 2-hydroxypropyl (meth)acrylate, tetrahydrofuryl acrylate, 1,6-hexanediol diacrylate, isobornyl acrylate, trimethylolpropane triacrylate, tri(propylene glycol) diacrylate, 1,2-ethanediol dimethacrylate, tris (2-hydroxy ethyl) isocyanurate triacrylate, 2-hydroxyethyl methacrylate, 2-hydroxyethyl acrylate, ethoxylated bisphenol A diacrylate, ethoxylated(3) trimethylolpropane tri(meth)acrylate, 2-(2-ethoxyethoxy) ethyl acrylate, dipentaerythritol hexaacrylate, and a combination thereof.

### [Initiator]

The initiator of the present disclosure is not particularly limited, and can be a suitable initiator known by those skilled in the art. The formulation of the present disclosure may comprise one or more initiators, such as radiation curable initiators and/or thermally curable initiators. For radiation curable initiators (e.g., photoinitiators), radiation curing is accomplished by exposure to high-energy radiation, e.g., UV radiation, or electron beams. The radiation dose typically sufficient for crosslinking in the case of UV curing is situated in the range from 80 to 3,000 mJ/cm², for example 1,500 to 2,000 mJ/cm². For thermal curable initiators, the curing temperature depends on the activation temperature of the thermal curable initiators used, typically ranging from 50°C to 250°C, but is limited by the thermal stability of the substrate. In some embodiments, a combination of both thermal and radiation curing may be used, and in other embodiments, curing may be accomplished by radiation curing alone.

Examples of suitable initiators include 1-[4-(2-Hydroxyethoxy)-phenyl]-2-hydroxy-2-methyl-1-propane-1-one (Irgacure 2959), 1-hydroxy-cyclohexyl-phenyl-ketone (Irgacure 184), 2-hydroxy-2-methyl-1-phenyl-propan-1-one (Irgacure 1173), ethyl (2,4,6-trimethylbenzoyl) phenyl phosphinate (TPO-L), 2,4,6-trimethylbenzoyl-diphenyl-phosphineoxide (TPO), methylbenzoylformate (MBF), benzoyl peroxide (BP), Agisyn^{™} 002 amine synergist, bis(2,4,6-trimethylbenzoyl)-phenylphosphineoxide (Irgacure 819), Esacure KIP 100 F, to name a few.

The content of the initiator is not particularly limited but can be adjusted depending on the content of the acrylic double bonds when needed. In one embodiment, the initiator may be added in the formulation in an amount of 1 to 10 parts by weight based on 100 parts by weight of the poly(meth)acrylate copolymer, for example, 1, 2, 3, 4, 5, 6, 7, 8, 9 or 10 parts by weight based on 100 parts by weight of the poly(meth)acrylate copolymer.

### [Formulation for Forming Poly(Meth)Acrylate Copolymer]

The formulation for forming the poly(meth)acrylate copolymer of the present disclosure can be prepared by any suitable method. In one embodiment, the oligomer may be used as is or may be premixed with a (meth)acrylic acid or (meth)acrylate monomer that is in a liquid state. In the latter case, the (meth)acrylic acid or (meth)acrylate monomer should be compatible with the oligomer. The compatibility can be determined, for example, by the appearance of the mixture of the oligomer and (meth)acrylic acid or (meth)acrylate monomer. If the mixture is transparent, it is considered that the oligomer and (meth)acrylic acid or (meth)acrylate monomer are compatible, and *vice versa.*

In one embodiment, the formulation is prepared by mixing one or more oligomers, one or more (meth)acrylic acids or (meth)acrylate monomers, and one or more initiators. In another embodiment, the formulation is prepared by mixing one or more oligomers that are premixed with a (meth)acrylic acid or (meth)acrylate monomer, one or more (meth)acrylic acids or (meth)acrylate monomers, and one or more initiators.

In some embodiments, the acrylate-functioned oligomer is present in the formulation in an amount of 10 to 75 parts by weight, or in an amount of 10 to 80 parts by weight based on the total weight of the formulation, e.g., 10 parts by weight, 15 parts by weight, 20 parts by weight, 25 parts by weight, 30 parts by weight, 35 parts by weight, 40 parts by weight, 45 parts by weight, 50 parts by weight, 55 parts by weight, 60 parts by weight, 65 parts by weight, 70 parts by weight, 75 parts by weight or 80 parts by weight.

In some embodiments, the (meth)acrylic acid or (meth)acrylate monomer is present in the formulation in an amount of 20 to 85 parts by weight, or in an amount of 20 to 90 parts by weight based on the total weight of the formulation, e.g., 20 parts by weight, 25 parts by weight, 30 parts by weight, 35 parts by weight, 40 parts by weight, 45 parts by weight, 50 parts by weight, 55 parts by weight, 60 parts by weight, 65 parts by weight, 70 parts by weight, 75 parts by weight, 80 parts by weight, 85 parts by weight or 90 parts by weight.

In some embodiments, the initiator is present in the formulation in an amount of 0.1 to 10 parts by weight based on the total weight of the formulation, e.g., 0.1 parts by weight, 0.2 parts by weight, 0.3 parts by weight, 0.5 parts by weight, 1 parts by weight, 2 parts by weight, 3 parts by weight, 4 parts by weight, 5 parts by weight, 6 parts by weight, 7 parts by weight, 8 parts by weight, 9 parts by weight or 10 parts by weight.

### [Substrate]

There is no special limitation to the species of the substrate for the present invention. In one embodiment of the present disclosure, the substrate can be a plastic substrate, a glass substrate, or a metal substrate, wherein the plastic substrate may be formed from a polymer, a copolymer, or a polymer mixture. For example, the substrate may be polyethylene terephthalate (PET) or polyethylene naphthalate (PEN); polymethacrylate resins, such as polymethyl methacrylate (PMMA); polyimide resins; polycycloolefin resins; polyolefin resins; polycarbonate resins; polyurethane resins; polystyrene resins; polyester resins, or a combination thereof. Preferably, the substrate is polyethylene terephthalate.

The thickness of the substrate is not particular limited. Generally, the thickness of the substrate may range from 50 µm to 200 µm, e.g., 50 µm, 60 µm, 70 µm, 80 µm, 90 µm, 100 µm, 110 µm, 120 µm, 130 µm, 140 µm, 150 µm, 160 µm, 170 µm, 180 µm, 190 µm or 200 µm. In one embodiment, the substrate is polyethylene terephthalate and has a thickness of 98.5 µm. In another embodiment, the substrate is polyethylene terephthalate and has a thickness of 100 µm.

### [Film]

The coating formed from the formulation of the present invention can be achieved by any means standard in the art, such as gravure, comma, reverse roll nip, reverse roll pan, hot melt, Mayer bar, knife over roll, slot orifice, immersion/dip coating, curtain coating, brushing, roll-to-roll coating, spray coating, spin coating, flow coating, and the like. The thickness of the coating of the present invention may range from 0.5 µm to 5 µm, from 1 µm to 3 µm, from 1.5 µm to 2 µm, or from 1.5 µm to 1.8 µm. The total thickness of the film (i.e., the coating along with the substrate on which it is formed) mainly depends on the thickness of the substrate.

The film may be used in a biological test strip, such as a blood glucose test strip, a uric acid test strip and other suitable test strips. The biological test strip may be a multi-function biological test strip which can be used to test more than one biological data. In some embodiments, the biological test strip may include at least (from bottom to top): an insulated substrate, a carbon layer/silver layer (which forms two working electrodes and one counter electrode), an insulation layer, a layer for testing biological data (for example, an enzyme layer for testing blood glucose, etc.), and a cover sheet (i.e., the film of the present disclosure).

To show a pleasant, appealing appearance, the film prepared according to the present invention should have good light transmittance, e.g., at least 80%, at least 85%, at least 88% or at least 90% light transmittance. As used herein, the term "transmittance" refers to the amount of light that passes through a coating or film divided by the total amount of light incident upon the sample. In addition, the film should have a low haze ratio, e.g., at most 5%, at most 4%, at most 3% or at most 2%. Furthermore, the film should have a low yellowness index, e.g., lower than 5, lower than 4, lower than 3 or lower than 2. Still further, the film should have a low average surface roughness, e.g., at most 0.05 µm, at most 0.04 µm, at most 0.03 µm, at most 0.02 µm.

To allow the body liquid, e.g., blood, saliva or urine, to diffuse quickly into a test area of a biological test strip, the film should have a low water contact angle. In addition, the film should have good wetting ability, e.g., fast diffusion rate of water and/or long total diffusion distance of water.

In contrast to commercial hydrophilic films, the film prepared according to the present disclosure does not contain a surfactant additive, but contains hydrophilic segments or hydrophilic groups in the backbone or at the end of the poly(meth)acrylate copolymer. The film prepared according to the present disclosure surprisingly has an enhanced hydrophilicity (including low water contact angle, fast diffusion rate of water and/or long total diffusion distance of water), which is superior to the conventional or commercial hydrophilic films, while exhibiting excellent stability and other properties. In some embodiments, the film prepared according to the present disclosure has a low water contact angle e.g., lower than 10°, lower than 9°, lower than 8°, lower than 7° or lower than 6°. In some embodiments, the film prepared according to the present disclosure has a superior diffusion rate of water, for example, the diffusion diameter of water diffused within three seconds is at least 5.25 mm, at least 5.50 mm, at least 5.75 mm, at least 6.00 mm or at least 6.25 mm. In some embodiments, the film prepared according to the present disclosure exhibits a superior total diffusion diameter of water than commercial hydrophilic films.

Amounts, ratios, and other numerical values are sometimes presented herein in a range format. It can be understood that such range formats are used for convenience and brevity, and should be understood flexibly to include not only numerical values explicitly specified as limits of a range, but also all individual numerical values or sub-ranges encompassed within that range as if each numerical value and sub-range is explicitly specified.

While the present disclosure has been described and illustrated with reference to specific embodiments thereof, these descriptions and illustrations do not limit the present disclosure. It can be clearly understood by those skilled in the art that various changes may be made, and equivalent elements may be substituted within the embodiments without departing from the true spirit and scope of the present disclosure as defined by the appended claims. The illustrations may not necessarily be drawn to scale. There may be distinctions between the artistic renditions in the present disclosure and the actual apparatus, due to variables in manufacturing processes and such. There may be other embodiments of the present disclosure which are not specifically illustrated. The specification is to be regarded as illustrative rather than restrictive. Modifications may be made to adapt a particular situation, material, composition of matter, method, or process to the objective, spirit and scope of the present disclosure. All such modifications are intended to be within the scope of the claims appended hereto. While the methods disclosed herein have been described with reference to particular operations performed in a particular order, it can be understood that these operations may be combined, sub-divided, or re-ordered to form an equivalent method without departing from the teachings of the present disclosure. Therefore, unless specifically indicated herein, the order and grouping of the operations are not limitations of the present disclosure.

### EXAMPLES

Embodiments of the present disclosure will now be further illustrated with reference to the following working examples and comparative examples, though without restricting its scope to these embodiments.

### Preparation Example 1: Synthesis of oligomer HEMA-IPDI-E02000-IPDI-HEMA

A 250 mL reactor (equipped with a stirrer, air inlet, dropping funnel, and condenser) was charged with 41.01 g of isophorone diisocyanate (IPDI), and was heated to 40°C, followed by purging with dry clean air. Then, 24.02 g of 2-hydroxyethyl methacrylate (HEMA) and 0.12 g of butylated hydroxytoluene (BHT) were charged into the reactor whilst stirring, followed by charging 0.12 g of TIB KAT^{®}716 manufactured by TIB Chemicals (a liquid catalyst based on bismuth metal). The mixture was agitated for reaction at the temperature of 40°C for one hour, and at an elevated temperature of 60°C for two additional hours. The quantity of isocyanate (NCO) content was measured by a potentiometric titrator to ensure it was within 11.89% of the value of the theoretical isocyanate content that would be derivable for the HEMA-IPDI intermediate from the quantities specified above. Upon confirmation of the appropriate isocyanate content, 184.73 g of polyol EO2000 purchased from Merck was added to the reactor. The resulting mixture was reacted at a temperature of 80°C for one hour. After this, the NCO content was checked via potentiometric titration again. The reaction was considered finished once the isocyanate content was lower than 0.1% relative to the entire weight of the composition, and was allowed to cool down to yield the subject oligomer, which has a viscosity of 1,998 mPas at 60°C. APHA color=15.

### Preparation Example 2: Synthesis of oligomer HEMA-IPDI-E03400-IPDI-HEMA

A 250 mL reactor (equipped with a stirrer, air inlet, dropping funnel, and condenser) was charged with 27.02 g of isophorone diisocyanate (IPDI), and was heated to 40°C, followed by purging with dry clean air. Then, 15.82 g of 2-hydroxyethyl methacrylate (HEMA) and 0.12 g of butylated hydroxytoluene (BHT) were charged into the reactor whilst stirring, followed by charging 0.12 g of TIB 716. The mixture was agitated for reaction at the temperature of 40°C for one hour, and at an elevated temperature of 60°C for two additional hours. The quantity of isocyanate (NCO) content was measured by a potentiometric titrator to ensure it was within 11.86% of the value of the theoretical isocyanate content that would be derivable for the HEMA-IPDI intermediate from the quantities specified above. Upon confirmation of the appropriate isocyanate content, 206.91 g of polyol EO3400 purchased from Merck was added to the reactor. The resulting mixture was reacted at a temperature of 80°C for one hour. After this, the NCO content was checked via potentiometric titration again. The reaction was considered finished once the isocyanate content was lower than 0.1% relative to the entire weight of the composition, and was allowed to cool down to yield the subject oligomer, which has a viscosity of 4,744 mPas at 60°C. APHA color=15.

### Preparation Example 3: Synthesis of oligomer HEMA-IPDI-E06000-IPDI-HEMA

A 250 mL reactor (equipped with a stirrer, air inlet, dropping funnel, and condenser) was charged with 16.54 g of isophorone diisocyanate (IPDI), and was heated to 40°C, followed by purging with dry clean air. Then, 9.69 g of 2-hydroxyethyl methacrylate (HEMA) and 0.12 g of butylated hydroxytoluene (BHT) were charged into the reactor whilst stirring, followed by charging 0.12 g of TIB 716. The mixture was agitated for reaction at the temperature of 40°C for one hour, and at an elevated temperature of 60°C for two additional hours. The quantity of isocyanate (NCO) content was measured by a potentiometric titrator to ensure it was within 11.82% of the value of the theoretical isocyanate content that would be derivable for the HEMA-IPDI intermediate from the quantities specified above. Upon confirmation of the appropriate isocyanate content, 223.52 g of polyol EO6000 purchased from Merck was added to the reactor. The resulting mixture was reacted at a temperature of 80°C for one hour. After this, the NCO content was checked via potentiometric titration again. The reaction was considered finished once the isocyanate content was lower than 0.1% relative to the entire weight of the composition, and was allowed to cool down to yield the subject oligomer, which has a viscosity of 13,295 mPas at 60°C. APHA color=20.

### Preparation Examples 4 to 7: Preparation of a formulation for forming a poly(meth)acrylate copolymer

From Preparation Examples 1 to 3, it can be seen that as the molecular weight of the oligomer increases, the viscosity also increases. Even though the oligomer HEMA-IPDI-E06000-IPDI-HEMA is expected to afford the highest hydrophilicity among the three oligomers due to its higher content of polyol, the ease of processing is a key consideration here, and the oligomer HEMA-IPDI-E02000-IPDI-HEMA obtained from Preparation Example 1 was used for the preparation of the formulation.

The oligomer HEMA-IPDI-E02000-IPDI-HEMA, 230T1 obtained from DSM (i.e., AgiSyn^{™} 230T1 of Covestro) (an aliphatic urethane acrylate oligomer), 2-(2-ethoxyethoxy) ethyl acrylate (EOEOEA), HEMA, tetrahydrofuryl acrylate (THFA), CN9013 manufactured by Sartomer (a multifunctional urethane acrylate oligomer), dipentaerythritol hexaacrylate (DPHA), 1-hydroxycyclohexyl phenyl ketone (Irgacure 184), and ethyl (2,4,6-trimethylbenzoyl) phenylphosphinate (TPO-L) were mixed according to the parts by weight shown in Table 1 as follows.

**Table 1**

| Content (wt%) | Preparation Example 4 | Preparation Example 5 | Preparation Example 6 | Preparation Example 7 |
|---|---|---|---|---|
| Oligomer HEMA-IPDI-EO 2000-IPDI-HEMA | 42 | 30 | 30 | 25 |
| 230T1 | - | - | 18 | - |
| EOEOEA | 25 | 30 | 9 | 25 |
| HEMA | 16 | 19 | 10 | 24 |
| THFA | 2 | 3 | 3 | 3 |
| CN9013 | 5 | 6 | 10 | 9 |
| DPHA | 10 | 12 | 15 | 14 |
| Irgacure 184 | 3 | 3 | 3 | 3 |
| TPO-L | 3 | 3 | 3 | 3 |

The appearance of the obtained formulations was transparent, indicating that the oligomer, acrylate monomer, and other components are compatible. All of the formulations in Preparation Examples 4 to 7 were found to reach a curing degree of >90%.

### Preparation of a hydrophilic film

The formulation for forming poly(meth)acrylate copolymer obtained in Preparation Example 4 was mixed with isopropyl alcohol (IPA) to form a kilo grade solution, and then roller-coated onto a PET film that was cut to a width of 450 mm. The roller coating was performed at ambient conditions.

### Test of the hydrophilic film

The physical properties of the hydrophilic film, including adhesion, pencil hardness, fingernail scratch property, average surface roughness, haze, storage property, transmittance, yellowness index, contact angle, and diffusion property were tested. The details of the tests are described below:

### 1. Adhesion

100 grid test was performed according to ASTM D-3359. The result was reported in a scale from ASTM class 5B to ASTM class 0B.

### 2. Pencil Hardness

A pencil for standard hardness test under a load of 1 kg was used to test the hardness of the coating. The hardness test was performed according to ASTM D3363.

### 3. Average Surface Roughness

The average surface roughness measurements were performed on a Taylor Hobson Co Ltd, Talysurf 50. The samples for the measurement were prepared by repeating the preparation of the hydrophilic film with the PET film cut to a width of 1020 mm.

### 4. Haze

Haze measurements were performed on a NIPPON DENSHOKU NDH 2000 haze meter.

### 5. Storage Test

The film was kept at a temperature of 27°C and a humidity of 60% for 30 days for storage stability measurements.

An accelerated storage test was performed by keeping the film in an oven at a temperature of 50°C for 30 days.

### 6. Transmittance & Yellowness Index

Transmittance and yellowness index measurements were performed on a SHIMADZU UV-3101PC UV/Vis/NIR Spectrophotometer with a wavelength range of 380 nm-700 nm.

### 7. Contact Angle

The contact angle was measured with an AST video contact angle (VCA) system. The volume of injected water was from 0.3 µL to 1 µL.

### 8. Diffusion Test (Wetting Test)

An Eppendorf Research^{®} plus pipette, 0.5-10 µL was used to sample 3 µL of DI water and inject the same onto the hydrophilic film. The circle size was observed within three seconds of the diffusion of the DI water. The symbol "⊚" indicates that the diffusion diameter is 6.00 mm (equivalent to wet index 44) or above. The symbol "○" indicates that the diffusion diameter is from 5.25 mm (equivalent to wet index 38) to 5.75 mm (equivalent to wet index 42). The symbol "×" indicates that the diffusion diameter is less than 5.25 mm.

The results of each test are shown in Table 2.

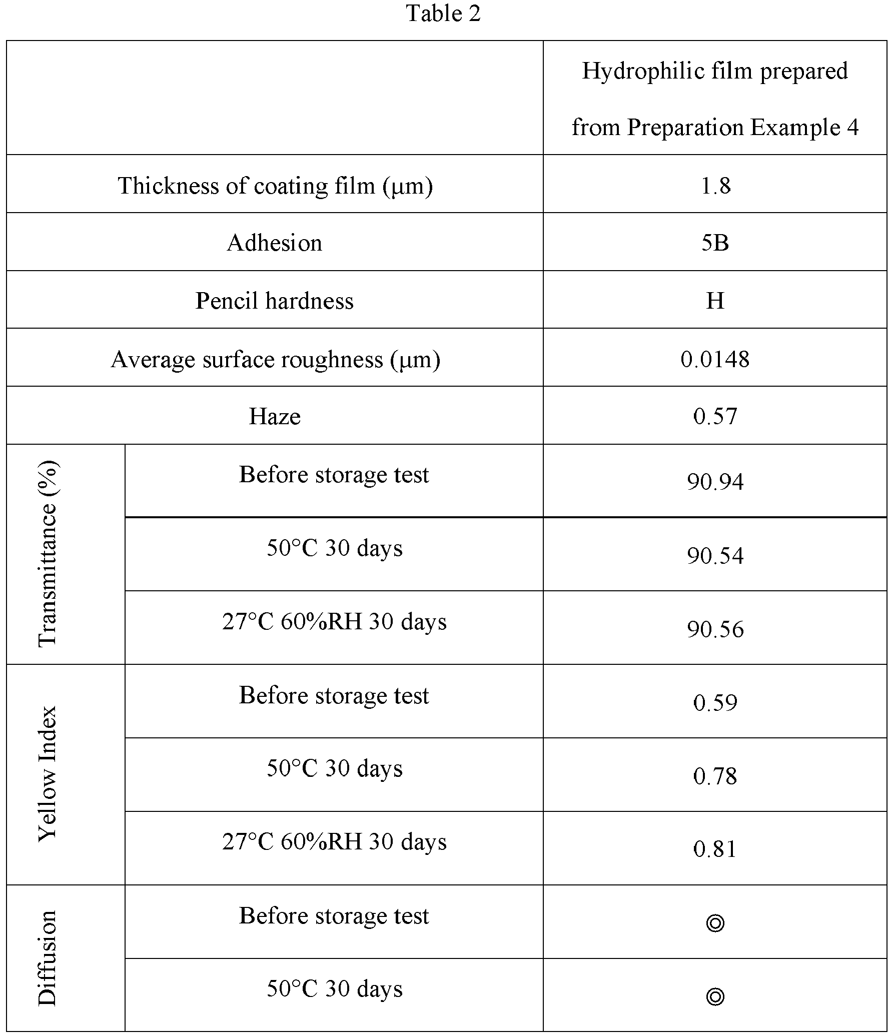

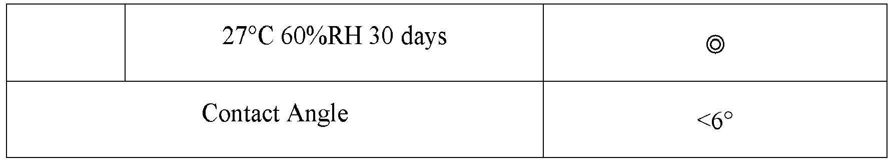

From the results it can be seen that the hydrophilic film prepared from the formulation of the present disclosure had good physical properties, such as adhesion, hardness, roughness, haze, storage stability, transmittance, yellowness, contact angle, wetting ability, etc. The hydrophilic films according to the present disclosure are suitable for use in biological test strips.

The above-described embodiments of the present disclosure are intended to be illustrative only. Numerous alternative embodiments may be devised by persons having ordinary skill in the art without departing from the scope of the following claims.

## Claims

1. A hydrophilic poly(meth)acrylate copolymer film for biological test strip, comprising:
a substrate; and
a poly(meth)acrylate copolymer formed on at least one surface of the substrate; wherein the poly(meth)acrylate copolymer is derived from a formulation comprising:
an acrylate-functioned oligomer;
a (meth)acrylic acid or (meth)acrylate monomer; and
an initiator.

2. The hydrophilic poly(meth)acrylate copolymer film according to Claim 1, wherein the acrylate-functioned oligomer is an acrylic acrylate oligomer, epoxy acrylate oligomer, polyester acrylate oligomer or urethane acrylate oligomer.

3. The hydrophilic poly(meth)acrylate copolymer film according to Claim 2, wherein the acrylate-functioned oligomer is urethane acrylate oligomer derived from:
a polyol;
a polyisocyanate; and
a hydroxyl-functioned (meth)acrylate.

4. The hydrophilic poly(meth)acrylate copolymer film according to Claim 3, wherein the polyol is selected from polyether polyol and polyester polyol and has a weight average molecular weight of 200 to 10,000.

5. The hydrophilic poly(meth)acrylate copolymer film according to Claim 3, wherein the polyisocyanate is selected from aliphatic polyisocyanate, cycloaliphatic polyisocyanate, araliphatic polyisocyanate, aromatic polyisocyanate, and a combination thereof, and contains two to six isocyanate groups.

6. The hydrophilic poly(meth)acrylate copolymer film according to Claim 3, wherein the hydroxyl-functioned (meth)acrylate is 2-hydroxyethyl methacrylate (HEMA) or 2-hydroxyethyl acrylate (HEA).

7. The hydrophilic poly(meth)acrylate copolymer film according to Claim 1, wherein the (meth)acrylate monomer comprises an ionic (meth)acrylate monomer or an non-ionic (meth)acrylate monomer.

8. The hydrophilic poly(meth)acrylate copolymer film according to Claim 7, wherein the ionic (meth)acrylate monomer is cationic, anionic or amphoteric.

9. The hydrophilic poly(meth)acrylate copolymer film according to Claim 1, wherein the (meth)acrylate monomer contains a hydrophilic repeating unit.

10. The hydrophilic poly(meth)acrylate copolymer film according to Claim 9, wherein the hydrophilic repeating unit is -(O-CH₂-CH₂)ₙ-, wherein n is 1 to 50.

11. The hydrophilic poly(meth)acrylate copolymer film according to Claim 1, wherein the (meth)acrylate monomer comprises a (meth)acrylate crosslinker containing two or more (meth)acrylate groups.

12. The hydrophilic poly(meth)acrylate copolymer film according to Claim 1, wherein the acrylate-functioned oligomer is in an amount of 10 to 80 parts by weight based on the total weight of the formulation.

13. The hydrophilic poly(meth)acrylate copolymer film according to Claim 1, wherein the (meth)acrylic acid or (meth)acrylate monomer is in an amount of 20 to 90 parts by weight based on the total weight of the formulation.

14. The hydrophilic poly(meth)acrylate copolymer film according to Claim 1, wherein the initiator is in an amount of 0.1 to 10 parts by weight based on the total weight of the formulation.

15. A method of preparing the hydrophilic poly(meth)acrylate copolymer film according to Claim 1, comprising applying the formulation to at least one surface of the substrate, and curing the formulation.

16. The method of Claim 15, wherein the formulation is applied by roller coating, gravure coating, gravure, comma, reverse roll nip, reverse roll pan, hot melt, Mayer bar, knife over roll, slot orifice, dip coating, curtain coating, brushing, spray coating, spin coating, or flow coating.

17. A biological test strip containing the hydrophilic poly(meth)acrylate copolymer film according to Claim 1.

18. The biological test strip according to Claim 17, which is a blood glucose test strip.
